# EUROPEAN PATENT SPECIFICATION

(11) **EP 2 559 421 B1**
(45) Date of publication and mention of the grant of the patent: **03.12.2014**
(21) Application number: 11177832.0
(22) Date of filing: 17.08.2011
(51) Int. Cl.: A61K 8/23, A61K 8/31, A61K 8/46, A61K 8/81, A61K 8/891, A61K 8/897, A61K 8/92, A61Q 9/04

(54) **Method and kit for depilation**
Verfahren und Kit zur Depilation
Procédé et kit d'épilation

(43) Date of publication of application: 20.02.2013
(73) Proprietor: The Procter & Gamble Company, Cincinnati, OH 45202 (US)
(72) Inventor: Smith, Paul, James, Whitton, Middlesex TW2 6EB (GB); Trani, Marina, Richmond, Surrey TW9 4BP (GB); Jakubovic, David, Andrew, Staines, Middlesex TW18 2DE (GB); Dring, Neil, Charles, Medmenham, Buckinghamshire SL7 2EZ (GB)
(74) Representative: Kohol, Sonia

(56) References cited:
- WO-A1-2011/119328
- WO-A2-2007/046097
- WO-A2-2007/119227
- WO-A2-2009/083836
- WO-A2-2011/119557
- WO-A2-2011/119794
- US-A- 2 202 829
- US-A1- 2004 219 118
- US-A1- 2005 031 547
- "Soothing Hair Removal Face Cream", GNPD; MINTEL, 1 May 2010 (2010-05-01), XP002665851, [retrieved on 2010-05-01]

## Description

### FIELD OF THE INVENTION

The present invention relates to a method and kit for depilation of skin.

### BACKGROUND OF THE INVENTION

Depilatory compositions are cosmetic hair removal formulations. They are generally aqueous compositions comprising keratin reducing agents, which reducing agents attack the disulphide bonds in hair to weaken it, such that subsequent gentle scraping and/or wiping completes severance of the hair from the skin and effects hair removal. Commercially, the most common keratin reducing agents are thioglycolates.

An unwanted side effect of chemical depilation is that, in addition to contacting hair to be removed, the depilatory composition comes into contact with skin. This skin contact combined with the alkaline conditions needed for effective hair removal may give rise to skin irritation.

US 2004/0219118 discusses the problem of skin irritation and proposes treatment with a "lipophilic" material before application of a thioglycolate-based reactive depilatory composition. The lipophilic materials exemplified in this patent application are oils, such as mineral oil.

### SUMMARY OF THE INVENTION

According to a first aspect of the invention, a method of removing hair from skin is provided, comprising the steps of:
(a) applying a depilatory composition to an area of skin on which unwanted hair is growing, the depilatory composition comprising a keratin reducing agent;
(b) subsequently applying a layer of liquid or semi-solid occlusive material to cover the depilatory composition, the liquid or semi-solid occlusive material comprising hydrophobic material, wherein the hydrophobic material comprises petrolatum, oils, waxes, silicones, fluorosilicones, fluoropolymers and triglycerides and mixtures thereof.

### DETAILED DESCRIPTION OF THE INVENTION

Applicants have established that the subsequent provision of layer of liquid or semi-solid occlusive material comprising hydrophobic material according to Claim 1, to cover a previously applied depilatory composition prevents water loss from the depilatory composition while the depilatory composition is in contact with the keratinous tissue and thus prevents the depilatory composition from drying out. Water loss from the depilatory composition lowers the water concentration, thereby increasing the concentration of active ingredients and bases present. Increasing the concentration of active ingredients and bases present may result in increased irritation to the skin.

In order to ensure that the liquid or semi-solid occlusive material performs its occlusive function throughout its application time, the volatility of the liquid or semi-solid occlusive material is advantageously such that less than 80%, preferably less than 65%, more preferably less than 60%, even more preferably less than 40% and more preferably still less than 20% by weight of the liquid or semi-solid occlusive material evaporates within 10 minutes from application under ambient conditions of 20°C and 65% relative humidity.

The liquid or semi-solid occlusive material preferably comprises from from 35% to 100%, preferably from 35% to 99%, more preferably from 40% to 95% and more preferably still from 50% to 95% of hydrophobic material by weight of the liquid or semi-solid occlusive material.

Suitable hydrophobic materials which may be comprised within the liquid or semi-solid occlusive material include petrolatum, oils, waxes, silicones, fluorosilicones, fluoropolymers, triglycerides and mixtures thereof.

If the hydrophobic material comprises an oil, then the oil is preferably selected from natural oil, synthetic oil, silicone oil and mixtures thereof.

Non-limiting examples of suitable natural oils include Acetylated Castor Oil, Acetylated Hydrogenated Castor Oil, Actinidia Chinensis (Kiwi), Seed Oil, Adansonia Digitata Oil, Aleurites Moluccana Seed Oil, Anacardium Occidentale (Cashew) Seed Oil, Arachis Hypogaea (Peanut) Oil, Arctium Lappa Seed Oil, Argania Spinosa Kernel Oil, Argemone Mexicana Oil, Avena Sativa (Oat) Kernel Oil, Bertholletia Excelsa Seed Oil, Borago Officinalis Seed Oil, Brassica Campestris (Rapeseed) Seed Oil, Calophyllum Tacamahaca Seed Oil, Camellia Japonica Seed Oil, Camellia Kissi Seed Oil, Camellia Oleifera Seed Oil, Canola Oil, Caprylic/Capric/Lauric Triglyceride, Caprylic/Capric/Linoleic Triglyceride, Caprylic/Capric/Myristic/Stearic Triglyceride, Caprylic/Capric/Stearic Triglyceride, Caprylic/Capric Triglyceride, Carthamus Tinctorius (Hybrid Safflower) Seed Oil, Carthamus Tinctorius (Safflower) Seed Oil, Carum Carvi (Caraway) Seed Oil, Carya IlIinoensis (Pecan) Seed Oil, Castor Oil Benzoate, Chenopodium Quinoa Seed Oil, Cibotium Barometz Oil, Citrullus Vulgaris (Watermelon) Seed Oil, Cocos Nucifera (Coconut) Oil, Cod Liver Oil, Coffea Arabica (Coffee) Seed Oil, Coix Lacryma-Jobi (Job's Tears) Seed Oil, Corylus Americana (Hazel) Seed Oil, Corylus Avellana (Hazel) Seed Oil, Cucumis Sativus (Cucumber) Oil, Cucurbita Pepo (Pumpkin) Seed Oil, Daucus Carota Sativa (Carrot) Seed Oil, Elaeis Guineensis (Palm) Kernel Oil, Elaeis Guineensis (Palm) Oil, Gossypium (Cotton) Seed Oil, Helianthus Annuus (Hybrid Sunflower) Oil, Helianthus Annuus (Sunflower) Seed Oil, Hippophae Rhamnoides Oil, Human Placental Lipids, Hydrogenated Canola Oil, Hydrogenated Castor Oil, Hydrogenated Castor Oil Laurate, Hydrogenated Castor Oil Triisostearate, Hydrogenated Coconut Oil, Hydrogenated Cottonseed Oil, Hydrogenated C12-18 Triglycerides, Hydrogenated Fish Oil, Hydrogenated Lard, Hydrogenated Menhaden Oil, Hydrogenated Mink Oil, Hydrogenated Olive Oil, Hydrogenated Orange Roughy Oil, Hydrogenated Palm Kernel Oil, Hydrogenated Palm Oil, Hydrogenated Peanut Oil, Hydrogenated Rapeseed Oil, Hydrogenated Shark Liver Oil, Hydrogenated Soybean Oil, Hydrogenated Sunflower Seed Oil, Hydrogenated Tallow, Hydrogenated Vegetable Oil, lsatis Tinctoria Seed Oil, Juglans Regia (Walnut) Seed Oil, Lauric/Palmitic/Oleic Triglyceride, Umnanthes Alba (Meadowfoam) Seed Oil, Unum Usitatissimum (Linseed) Seed Oil, Lupinus Albus Seed Oil, Macadamia Integrifolia Seed Oil, Macadamia Ternifolia Seed Oil, Maleated Soybean Oil, Mangifera Indica (Mango) Seed Oil, Marmot Oil, Melaleuca Alternifolia (Tea Tree) Leaf Oil, Melia Azadirachta Seed Oil, Melissa Officina lis (Balm Mint) Seed Oil, Menhaden Oil, Mink Oil, Moringa pterygosperma Seed Oil, Mortierella Oil, Neatsfoot Oil, Nelumbium Speciosum Flower Oil, Nigella Sativa Seed Oil, Oenothera Biennis (Evening Primrose) Oil, Olea Europaea (Olive) Fruit Oil, Olea Europaea (Olive) Husk Oil, Orange Roughy Oil, Orbignya Cohune Seed Oil, Orbignya Oleifera Seed Oil, Oryza Sativa (Rice) Bran Oil, Oryza Sativa (Rice) Germ Oil, Ostrich Oil, Oxidized Corn Oil, Oxidized Hazel Seed Oil, Papaver Orientale (Poppy) Seed Oil, Passiflora Edulis Seed Oil, Persea Gratissima (Avocado) Oil, Pistacia Vera Seed Oil, Placental Lipids, Prunus Amygdalus Amara (Bitter Almond) Kernel Oil, Prunus Amygdalus Dulcis (Sweet Almond) Oil, Prunus Armeniaca (Apricot) Kernel Oil, Prunus Avium (Sweet Cherry) Seed Oil, Prunus Cerasus (Bitter Cherry) Seed Oil, Prunus Persica (Peach) Kernel Oil, Pyrus Malus (Apple) Oil, Ribes Nigrum (Black Currant) Seed Oil, Ricinus Communis (Castor) Seed Oil, Rosa Canina Fruit Oil, Rosa Moschata Seed Oil, Salmon Oil, Salvia Hispanica Seed Oil, Santalum Album (Sandalwood) Seed Oil, Sesamum Indicum (Sesame) Seed Oil, Shark Liver Oil, Solanum Lycopersicum (Tomato) Seed Oil, Soybean Lipid, Sphingolipids, Taraktogenos Kurzii Seed Oil, Telphairia Pedata Oil, Vegetable Oil, Vitis Vinifera (Grape) Seed Oil, Zea Mays (Corn) Germ Oil, Zea Mays (Corn) Oil and mixtures thereof.

Non-limiting examples of suitable synthetic oils include mineral oil, isopropyl pamitate, isopropyl stearate, isohexadecane, isododecane, polyglyceryl triisostearate and mixtures thereof. Non-limiting examples of suitable silicone oils include dimethicones (including partial esters of dimethicones and fatty acids derived from natural/synthetic oils), cyclomethicones, polydimethlysiloxanes (e.g. DC200 from Dow Coming), phenyl trimethicones, trimethyl pentaphenyl trisiloxane, dimethicone copolyols and mixtures thereof.

If the hydrophobic material comprises a wax, then the wax is preferably selected from natural wax, synthetic wax, silicone wax, and mixtures thereof.

Non-limiting examples of suitable natural waxes include Abies Alba Leaf Wax, Acacia Dealbata Leaf Wax, Acacia Farnesiana Flower Wax, Beeswax, Ceresin, Cetyl Esters, Cistus Labdaniferus Flower Wax, Aurantium Amara (Bitter Orange) Flower Wax, Aurantium Dulcis (Orange) Peel Wax, Copernicia Cerifera (Carnauba) Wax, Eclipta Prostrata Wax, Euphorbia Cerifera (Candelilla) Wax, Helichrysum Angustifolium Wax, Jasminum Officina le (Jasmine) Flower Wax, Jasminum Sambac (Jasmine) Flower Wax, Jojoba Esters, Jojoba Wax, Lanolin Wax, Lavandula Angustifolia (Lavender) Flower Wax, Lawsonia Inermis Wax, Mink Wax, Montan Acid Wax, Montan Wax, Myrica Cerifera (Bayberry) Fruit Wax, Ocimum Tenuiflorum Wax, Olive Wax, Oryza Sativa (Rice) Bran Wax, Ouricury Wax, Palm Kernel Wax, Persea Gratissima (Avocado) Wax, Pistacia Lentiscus Leaf Wax, Polianthes Tuberosa Flower Wax, Pyrus Malus (Apple) Peel Wax, Ribes Nigrum (Black Currant) Wax, Rosa Centifolia Flower Wax, Salvia Sclarea (Clary) Wax, Shellac Wax, Simmondsia Chinensis (Jojoba) Butter, Soft Olive Wax, Spent Grain Wax, Stipa Tenacissima Wax, Sunflower Seed Wax, Vegetable Wax, Vitis Vinifera (Grape) Leaf Wax and mixtures thereof.

Non-limiting examples of suitable synthetic waxes include Hydrogenated Japan Wax, Hydrogenated Jojoba Oil, Hydrogenated Jojoba Wax, Hydrogenated Microcrystalline Wax, Hydrogenated Rice Bran Wax, Hydrolyzed Beeswax, Microcrystalline Wax, Oxidized Beeswax, Oxidized Microcrystalline Wax, Ozokerite, Paraffin, PEG-6 Beeswax, PEG-8 Beeswax, PE G-12 Beeswax, PEG-20 Beeswax, PEG-12 Carnauba, Potassium Oxidized Microcrystalline Wax, Sulfurized Jojoba Oil, Synthetic Beeswax, Synthetic Candelilla Wax, Synthetic Carnauba, Synthetic Japan Wax, Synthetic Jojoba Oil, Synthetic Wax and mixtures thereof.

Non-limiting examples of suitable silicone waxes include DC2503 Cosmetic Wax, DC580 wax, DC AMS-C30 Cosmetic Wax, C30-45 Alkyl Methicone, DC Silkywax 10, Hexamethyldisiloxane, DC ST-Wax 30, C30-45 Alkyldimethylsilyl Polypropylsilsesquioxane, DC SW-8005 resin wax, C26 - 28 Alkyl Dimethicone, C26 - 28 Alkyl Methicone, Polyphenylsilsesquioxane and mixtures thereof.

Advantageously, the wax comprises beeswax, carnauba wax, candelilla wax, jojoba wax, paraffin wax, microcrystalline wax, ozokerite, arachidyl behenate, or mixtures thereof.

If the hydrophobic material comprises a triglyceride, then the triglyceride shall have the following formula: 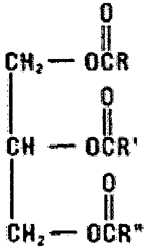 wherein R, R' and R" may be the same as or different from one or both of the others, wherein each of R, R' and R" is a fatty acid and wherein the triglyceride is solid at 25°C

Suitable oils from which triglycerides may be formed from include, but are not limited to, the oils listed above. Suitable fatty acids for formation of triglycerides include, but are not limited to, Myristoleic acid, Palmitoleic acid, Sapienic acid, Oleic acid, Linoleic acid, α-Linolenic acid ,Arachidonic acid , Eicosapentaenoic acid, Docosahexaenoic acid, Lauric acid (C₁₂), Myristic acid (C₁₄), Palmitic acid (C₁₆), Stearic acid (C₁₈), Arachidic acid (C₂₀) and mixtures thereof.

Specific sources of triglycerides suitable for inclusion in the liquid or semi-solid occlusive material include Butter, Shea Butter, Butyrospermum Parkii, Lipex Shea, Theobroma Cacao (Cocoa) Seed Butter, Cocoa Butter, Hydrogenated Shea Butter, Hydrogenated Cocoa Butter, Irvingia Gabonensis Kernel Butter, Tallow, Lard, Mangifera Indica (Mango) Seed Butter, Kokum Butter and mixtures thereof The triglyceride(s) may fall under the definition of "wax" or "oil" as used herein and, in such a case, should be included as a wax or oil for the purposes of determining the proportions of wax or oil.

The hydrophobic material comprised within the liquid or semi-solid occlusive material may additionally comprise skin active agents such as, but not limited to oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils and mixtures thereof.

The liquid or semi solid occlusive material may comprise an emulsion. The emulsion may comprise a continuous hydrophilic phase such as a silicone-in-water emulsion, an oil-in-water emulsion, and a water-in-oil-in-water emulsion. Alternatively, the emulsion may comprise continuous hydrophobic phase, such as a water-in-oil emulsion, a water-in-silicone emulsion and a oil-in-water-in-silicone emulsion. The emulsion may further contain an emulsifier. The emulsifier may be present from 0.1% to 15%, by weight of the liquid or semi-solid occlusive material, preferably from 0.2% to 10%, and even more preferably from 0.5% to 8%. Emulsifiers may be nonionic, anionic or cationic. Suitable emulsifiers are disclosed in, for example, U.S. Patent 3,755,560, U.S. Patent 4,421,769, and McCutcheon's Detergents and Emulsifiers, North American Edition, pages 317-324 (1986).

The liquid or semi-solid occlusive material may additionally comprise a thickening agent. A representative but not exhaustive list can be found in "The Encyclopaedia of Polymers and Thickeners for Cosmetics" compiled and edited by Robert Y. Lochhead, PhD and William R30 Fron, Department of Polymer Science, University of Southern Mississippi. Non-limiting examples of suitable thickening agents include gums, carbomers, polymers and copolymers of acrylic acid, associated thickeners, layered silicates/clays and natural polymers (including polysaccharides). One or more thickening agents may be included in the liquid or semi-solid occlusive material. The thickening agent may be present at a level of from 0.01 % to 20%, preferably from 0.1% to 15%, more preferably from 0.3% to 10%, and even more preferably from 0.5% to 6%, by weight of the occlusive liquid or semi-solid.

The liquid or semi-solid occlusive material may comprise one or more particulates which serve as powders or fillers. Non-limiting examples of suitable particulates include fumed silica, chalk, hectorite, silica, kaoline, nylon, polypropylene, polystyrene, calcium carbonate, calcium silicate, alumina, mico, cellulose, corn starch. Suitable levels of particulates may be from 0.1% to 60%, preferably from 0.5% to 40%, more preferably from 1.0% to 30% and even more preferably from 2.0% to 20%, by weight of the liquid or semi-solid occlusive material.

Preferably, the liquid or semi-solid occlusive material is transparent. As used herein, the term "transparent" refers to materials, articles and/or compositions through which it is possible to see with the naked eye. This enables the user to see unwanted hairs being treated by the depilatory composition and therefore visually to assess progress being made by the depilatory activity rather than relying on estimated timings alone. This aids the user in improving depilatory efficacy while reducing the risk of skin irritation.

Advantageously, the liquid or semi-solid occlusive material may comprise inorganic or organic pigments or dyes in order to colour the liquid or semi-solid occlusive material and enable the user readily to distinguish the areas of the depilatory composition that have been covered by the liquid or semi-solid occlusive material from those that have not been covered. Alternatively, the depilatory composition may be coloured or alternatively again, both the liquid or semi-solid occlusive material and the depilatory composition may be coloured. Preferably, the liquid or semi-solid occlusive material is both coloured and transparent, such that the depilatory composition and unwanted hair is clearly visible yet distinguishable in use. Typical hair colours are white, grey, black, brown, red (ginger) and blonde. Accordingly, in a preferred embodiment, the liquid or semi-liquid occlusive material is coloured so as not to match typical hair colours. Advantageously, the selected colour may be green, blue, purple (violet) or pink. Non-limiting examples of suitable colourants may be inorganic or organic, including D&C and FD&C blue, brown and yellows etc, iron oxides, ultramarines and chromium hydroxide colours. Suitable levels of inorganic or organic pigments may be from 0.01% to 10%, preferably from 0.01 to 5%, and more preferably from 0.05% to 3%, by weight of the liquid or semi-solid occlusive material.

The liquid or semi-solid occlusive material may also comprise a fragrance or an odor masking agent to reduce the malodor that arises from the typically sulphur-containing keratin reducing agents that provide activity to the depilatory composition. The fragrance or odor masking agent may comprise an aldehyde, a lactone, a ketone, an alcohol, a terpene, an ester or a mixture thereof and be present from 0.01 % to 8%, by weight of the liquid or semi-solid occlusive material, more preferably from 0.05% to 5%, and even more preferably from 0.1% to 3%

The liquid or semi-solid occlusive material may include further ingredients such as, but not limited to skin care ingredients, and depilatory enhancers and mixtures thereof, provided that their presence does not impede the ability of the liquid or semi-solid occlusive material to occlude the depilatory composition.

Any depilatory composition comprising a suitable keratin reducing agent may be used in the present method and included in the present kit. Non-limiting examples of suitable keratin reducing agents include: sulphide salts such as Li₂S, Na₂S, K₂S, MgS, CaS, SrS or BaS, hydrogen sulphide salts such as NaSH or KSH; thioglycol; thioglycerol; thioglycolamide; thioglycolhydrazide; thioglycolic acid; thioglycolate salts (such as potassium thioglycolate, calcium thioglycolate, ammonium thioglycolate, diammonium dithioglycolate, glyceryl monothioglycolate, or monoethanolamine thioglycolate); thiosalicylic acid; thiomalic acid; ammonium thiolactate; monoethanolamine thiolactate; dithioerythritol; 2-mercaptopropionic acid; 1,3-dithiopropanol; glutathione; dithiothreitol; cysteine; homocysteine; N-acetyl-L-cysteine and cysteamine. Advantageously, the keratin reducing agent is comprised within the depilatory composition in an amount from 0.3% to 20%, preferably from 0.8% to 15%, more preferably from 1% to 10% by weight of the depilatory composition.

Advantageously, the depilatory composition may comprise at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof

The pH of the depilatory composition may advantageously be in the range of from 6 to 13.8, preferably from greater than 7 to 13, more preferably from 9 to 12.9, even more preferably from 10 to 12.8, even more preferably still from 12 to 12.75 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide, sodium hydroxide or mixtures thereof.

In an advantageous embodiment, the base is present at a concentration of from 0.1% to 10.0%, more preferably from 0.5% to 8.0% and even more preferably from 1.0% to 5.0%, by weight of the depilatory composition.

The concentration of water in the depilatory composition is preferably at least 40%, more preferably from 50% to 98%, even more preferably from 60% to 95% and even more preferably still from 70% to 90%, by weight of the depilatory composition (and such shall be referred to hereafter as "aqueous depilatory composition").

The aqueous depilatory composition may comprise at least one monovalent cation, preferably a monovalent metal cation. The monovalent cation(s), such as those derived from monovalent cation-containing salts, are able to displace the cation of the thioglycolate salt and further enhance dissociation of said thioglycolate salt. This increases the amount of deprotonated thioglycolate formed from the thioglycolate salt and therefore increases the effectiveness of the aqueous depilatory composition. Sources of monovalent cations include potassium, sodium, lithium, ammonium, tetraalkyl ammonium and imidazolium salts, which may be a component of another ingredient, for example a thickening system or skin care active. Preferred sources of monovalent cations include potassium and sodium salts.

The quantity of monovalent cations (or in a preferred embodiment above monovalent metal cations) per unit area of the area of skin to which the depilatory composition has been applied is from 6 × 10⁻⁷ mol/cm² to 6 × 10⁻⁶ mol/cm², preferably from 1.5 × 10⁻⁶ mol/cm² to 4.5 × 10⁻⁶ mol/cm² and more preferably from 2.25 × 10⁻⁶ mol/cm² to 3.75 × 10⁻⁶ mol/cm². Without wishing to be bound by theory, the applicants believe that within this narrow range of monovalent cation dosage, the efficacy of the depilatory composition may be increased while remaining within the bounds of tolerance of human skin, consequently reducing irritation. The selection of keratin reducing agent and optional ingredients including the base may be made considering the quantity of monovalent cations or monovalent metal cations achieved.

The depilatory composition may optionally comprise a thickening agent. A representative but not exhaustive list can be found in "The Encyclopaedia of Polymers and Thickeners for Cosmetics", referred to above. Exemplary classes of thickening agents include gums, carbomers, polymers and copolymers of acrylic acid, associated thickeners, layered silicates/clays and natural polymers (including polysaccharides). One or more thickening agents may be included in the aqueous depilatory composition. The thickening agent may be present at a level of from about 0.01% to about 20%, preferably from about 0.1 % to about 10% by weight of the depilatory composition.

Advantageously, the depilatory composition may have a yield point from 10 Pa to 2000 Pa, more preferably from 30 Pa to 1200 Pa, even more preferably from 45 Pa to 500 Pa and even more preferably still from 60 Pa to 300 Pa, when measured via a stress controlled amplitude sweep at a frequency of 1 Hz and a temperature of 25°C. The yield point described is defined as the 5% decrease in magnitude of the elastic modulus G' linear viscoelastic plateau value as measured on a TA1000 Rheometer, available from TA Instruments of New Castle, Delaware, USA. The rheological properties of the depilatory composition may be altered by changing the concentration or identity of the thickening system and the water content of the depilatory composition.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone, including retinoids), cosmetic compositions; anti-inflammatory agents (including corticosteroids); anti-oxidants (including flavonoids) radical scavengers; sunscreen agents; skin cooling or warming agents and the like. The depilatory composition may comprise one or more skin care ingredients in an amount of from about 0.001% to about 10%, more preferably from about 0.01% to about 7%, and even more preferably from about 0.025% to about 5%, by weight of the depilatory composition.

An accelerant may be employed in the depilatory composition, which accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, preferably from 2% to 8% and more preferably from 2% to 5% by weight of the depilatory composition.

The depilatory composition may further comprise components known, conventionally used, or otherwise effective for use in cosmetic compositions, such as dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

The depilatory composition may be formulated in any common delivery form, such as a gel, cream or lotion.

Prior to applying the method or using the kit according to the present invention, a user should advantageously remove all make-up from the skin, to ensure good adherence and effective application of both the depilatory composition and the liquid or semi-solid occlusive material.

Preferably, the depilatory composition is applied to the skin in an amount per unit area from 0.001 g/cm² to 0.6 g/cm², more preferably from 0.003 g/cm² to 0.5 g/cm², even more preferably from 0.005 g/cm² to 0.3 g/cm² and even more preferably still from 0.2 g/cm² to 0.01 g/cm². The depilatory composition may be applied to the skin by any means, including the user's finger or hand, from the depilatory composition container such as a tube, bottle or sachet, via a tool (including spatulas, pipettes, brushes, sponges, tines, foams, non-wovens, wovens sintered elements and pads) or dispensed via an applicator (including rollers, sprays / atomizers, pistons, pumps, pressurized containers, tilt valves and integrated devices).

Subsequent to, but as soon as possible after application of the depilatory cream, the layer of liquid or semi-solid occlusive material is applied. The liquid or semi-solid occlusive material should be applied such that the surface of the depilatory composition is fully covered and may extend beyond the perimeter edge of the depilatory composition, advantageously by up to 8 mm, preferably up to 5 mm and more preferably up to 2 mm.

The liquid or semi-solid occlusive material may be applied onto the depilatory composition by any means, including the user's finger or hand, directly from a container such as a tube, bottle or sachet, via a tool (including spatulas, pipettes, brushes, sponges, tines, foams, non-wovens, wovens sintered elements and pads) or dispensed via an applicator (including rollers, sprays/atomizers, pistons, pumps, pressurized containers, tilt valves and integrated devices). Preferably, the liquid or semi-solid occlusive material is applied by an applicator (including rollers, sprays/atomizers, pistons, pumps, pressurized containers, tilt valves or integrated devices).

Preferably, the liquid or semi-solid occlusive material is applied to the depilatory composition in an amount per unit area of from 0.001 g/cm² to 0.6 g/cm², more preferably from 0.003 g/cm² to 0.5 g/cm², even more preferably from 0.005 g/cm² to 0.3 g/cm² and more preferably still from 0.01 g/cm² to 0.2 g/cm² . The liquid or semi-solid occlusive material is advantageously applied as a continuous layer which completely covers the surface of the depilatory composition.

Following application, the depilatory composition and the layer of liquid or semi-solid occlusive material are advantageously left in place for at least 1 minute, preferably from 1 to 10 minutes, more preferably from 3 to 10 minutes, depending on the thickness of the hair and the hair removal efficacy of the depilatory composition (which, in turn, is dependent upon the concentration of keratin reducing agent in the depilatory composition).

Once the depilatory composition has been in place for sufficient time to be effective, then it and the layer of liquid or semi-solid occlusive material are advantageously removed. Removal may be achieved using one or more of a cotton wool ball, pad or wand, a tissue, a cloth, or a tool, such as a spatula or a scraper.

Advantageously, the skin from which hair has been removed is then rinsed with water.

In an advantageous subsequent step, a post-treatment skin care composition may be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

### Examples

Depilatory composition example:

| Depilatory Ingredient | % w/w |
|---|---|
| DI water | 84.42 |
| Acrylic acid / VP crosspolymer (Ultrathix P-100)¹ | 3.00 |
| Calcium hydroxide² | 4.50 |
| Calcium thioglycolate trihydrate³ | 6.00 |

| | |
|---|---|
| 1 Ultrathix P-100 available from International Specialty Products Inc. (ISP) 2 Calcium hydroxide Reag. Ph. Eur. puriss. p.a. available from Sigma-Aldrich Co. 3 Calcium thioglycolate trihydrate 99.8% available from BRUNO BOCK Chemische Fabrik GmbH & Co. | |

### Occlusive liquid examples:

| Ingredient | % w/w | % w/w |
|---|---|---|
| Silicone Oil DC-200¹ | 100 | n/a |
| PEG-35 hydrogenated castor oil² | n/a | 100 |

| | | |
|---|---|---|
| ¹ Silicone Oil DC-200 available from Dow Corning. ² PEG-35 hydrogenated castor oil available from Sigma-Aldrich Co. | | |

The depilatory composition was applied to the skin of the outer forearm (1.5cm in width and 3.5cm in length and 2mm in thickness). The liquid occlusive material was applied onto the depilatory composition such that the liquid extended continuously over the depilatory composition and beyond it by 2mm at every point on the perimeter of the applied depilatory composition to prevent water from being lost from the depilatory composition during the application period.

## Claims

1. A method of removing hair from skin, preferably facial skin, comprising the steps of:
(a) applying a depilatory composition to an area of skin on which unwanted hair is growing, the depilatory composition comprising a keratin reducing agent;
(b) subsequently applying a layer of liquid or semi-solid occlusive material to cover the depilatory composition, the liquid or semi-solid occlusive material comprising hydrophobic material, wherein the hydrophobic material comprises petrolatum, oils, waxes, silicones, fluorosilicones, fluoropolymers and triglycerides or mixtures thereof.

2. The method of claim 1, wherein the liquid or semi-solid occlusive material comprises from 35% to 100%, preferably from 35% to 99%, more preferably from 40% to 95% and more preferably still from 50% to 95% of hydrophobic material by weight of the liquid or semi-solid occlusive material.

3. The method of any preceding claim, wherein the liquid or semi-solid occlusive material is applied in an amount per unit area of from 0.001 g/cm² to 0.6 g/cm², preferably from 0.003 g/cm² to 0.5 g/cm², more preferably from 0.005 g/cm² to 0.3 g/cm² and more preferably still from 0.01 g/cm² to 0.2 g/cm² .

4. The method of any preceding claim, wherein the liquid or semi-solid occlusive material is applied as a continuous layer which completely covers the depilatory composition.

5. The method of any preceding claim, wherein the layer of liquid or semi-solid occlusive material is applied by painting or spraying.

6. The method of any preceding claim, wherein the keratin reducing agent comprises a thioglycolate salt, preferably potassium or calcium thioglycolate, or mixtures thereof.

## Patentansprüche

1. Verfahren zum Entfernen von Haaren von der Haut, vorzugsweise Gesichtshaut, das die folgenden Schritte umfasst:
(a) Aufbringen einer Enthaarungszusammensetzung auf einen Hautbereich, auf dem unerwünschte Haare wachsen, wobei die Enthaarungszusammensetzung ein Keratinreduktionsmittel umfasst,
(b) nachfolgendes Aufbringen einer Schicht eines flüssigen oder halbfesten okklusiven Materials, um die Enthaarungszusammensetzung abzudecken, wobei das flüssige oder halbfeste okklusive Material hydrophobes Material umfasst, wobei das hydrophobe Material Petrolatum, Öle, Wachse, Silikone, Fluorsilikone, Fluorpolymere und Triglyceride oder Mischungen davon umfasst.

2. Verfahren gemäß Anspruch 1, wobei das flüssige oder halbfeste okklusive Material 35 Gew.-% bis 100 Gew.%, vorzugsweise 35 Gew.-% bis 99 Gew.-%, mehr bevorzugt 40 Gew.-% bis 95 Gew.-% und noch mehr bevorzugt 50 Gew.-% bis 95 Gew.-% eines hydrophoben Materials, bezogen auf das Gewicht des flüssigen oder halbfesten okklusiven Materials, umfasst.

3. Verfahren nach einem der vorstehenden Ansprüche, wobei das flüssige oder halbfeste okklusive Material in einer Menge pro Einheitsfläche von 0,001 g/cm² bis 0,6 g/cm², vorzugsweise von 0,003 g/cm² bis 0,5 g/cm², mehr bevorzugt von 0,005 g/cm² bis 0,3 g/cm² und noch mehr bevorzugt von 0,01 g/cm² bis 0,2 g/cm² aufgebracht wird.

4. Verfahren nach einem der vorstehenden Ansprüche, wobei das flüssige oder halbfeste okklusive Material als ununterbrochene Schicht aufgebracht wird, welche die Enthaarungszusammensetzung vollständig abdeckt.

5. Verfahren nach einem der vorstehenden Ansprüche, wobei das flüssige oder halbfeste okklusive Material durch Aufpinseln oder Aufsprühen aufgebracht wird.

6. Verfahren nach einem der vorstehenden Ansprüche, wobei das Keratinreduktionsmittel ein Thioglycolatsalz, bevorzugt Kalium- oder Calciumthioglycolat, oder Mischungen davon umfasst.

## Revendications

1. Procédé d'élimination des poils de la peau, de préférence la peau du visage, comprenant les étapes consistant à :
(a) appliquer une composition dépilatoire sur une zone de peau sur laquelle poussent des poils indésirables, la composition dépilatoire comprenant un agent réducteur de kératine ;
(b) appliquer ultérieurement une couche de matériau occlusif liquide ou semi-solide afin de couvrir la composition dépilatoire, le matériau occlusif liquide ou semi-solide comprenant un matériau hydrophobe, ledit matériau hydrophobe comprenant de la vaseline, des huiles, des cires, des silicones, des fluorosilicones, des fluoropolymères et des triglycérides ou leur(s) mélange(s).

2. Procédé selon la revendication 1, dans lequel le matériau occlusif liquide ou semi-solide comprend de 35 % à 100 %, de préférence de 35 % à 99 %, plus préférablement de 40 % à 95 % et mieux encore de 50 % à 95 % de matériau hydrophobe en poids du matériau occlusif liquide ou semi-solide.

3. Procédé selon l'une quelconque des revendications précédentes, dans lequel le matériau occlusif liquide ou semi-solide est appliqué en une quantité par surface unitaire de 0,001 g/cm² à 0,6 g/cm², de préférence de 0,003 g/cm² à 0,5 g/cm², plus préférablement de 0,005 g/cm² à 0,3 g/cm² et encore plus préférablement de 0,01 g/cm² à 0,2 g/cm².

4. Procédé selon l'une quelconque des revendications précédentes, dans lequel le matériau occlusif liquide ou semi-solide est appliqué en une couche continue recouvrant complètement la composition dépilatoire.

5. Procédé selon l'une quelconque des revendications précédentes, dans lequel la couche de matériau occlusif liquide ou semi-solide est appliquée par peinture ou pulvérisation.

6. Procédé selon l'une quelconque des revendications précédentes, dans lequel l'agent réducteur de kératine comprend un sel de thioglycolate, de préférence du thioglycolate de potassium ou calcium, ou leurs mélanges.
